# EUROPÄISCHE PATENTSCHRIFT

(11) **EP 2 137 153 B1**
(45) Veröffentlichungstag und Bekanntmachung des Hinweises auf die Patenterteilung: **20.06.2012**
(21) Anmeldenummer: 08749024.9
(22) Anmeldetag: 21.04.2008
(51) Int. Cl.: C07D 213/06, C07D 213/79, C07D 213/81

(54) **VERFAHREN ZUR HYDROLYSE VON HETEROAROMATISCHEN NITRILEN IN WÄSSRIGEN FLUIDEN**
METHOD FOR THE HYDROLYSIS OF HETEROAROMATIC NITRILES IN AQUEOUS FLUIDS
PROCÉDÉ D'HYDROLYSE DE NITRILES HÉTÉRO-AROMATIQUES DANS DES FLUIDES AQUEUX

(30) Priorität: 20.04.2007 EP 07008087; 04.05.2007 EP 07009045
(43) Veröffentlichungstag der Anmeldung: 30.12.2009
(73) Patentinhaber: LONZA AG, 4052 Basel (CH)
(72) Erfinder: OTT, Lothar, CH-3930 Visp (CH); HEYL, Andreas, CH-3912 Termen (CH); CLAUSEN, Norbert, CH-3968 Veyras (CH); VOGEL, Herbert, 64569 Nauheim (DE); MICHALIK, Gregor, 64285 Darmstadt (DE)
(86) Internationale Anmeldenummer: PCT/EP2008/003186
(87) Internationale Veröffentlichungsnummer: WO 2008/128744

(56) Entgegenhaltungen:
- US-A- 2 446 957
- US-A- 4 008 241
- US-A- 4 314 064
- US-A- 5 756 750
- SUVOROV ET AL: "Hydrolysis of nitriles. IV. Continuous hydrolysis of 3- cyanopyridine to nicotinamide and nicotinic acid" STN CAPLUS, 1973, XP002127619
- SUVOROV ET AL: "Production of nicotinic acid from coke by-product.beta.-picoline" CHEMICAL ABSTRACTS + INDEXES, AMERICAN CHEMICAL SOCIETY. COLUMBUS, US, Bd. 19, Nr. 86, 9. Mai 1977 (1977-05-09), XP002096347 ISSN: 0009-2258

## Beschreibung

Die vorliegende Erfindung betrifft ein Verfahren zur Hydrolyse von Verbindungen der Formeln 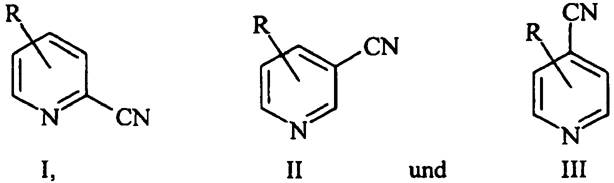 worin R Wasserstoff oder C₁₋₂₀-Alkyl ist, in wässrigen Fluiden unter Hochdruckbedingungen.

Heteroaromatische Nitrile der Formeln I oder II, insbesondere Nikotinsäurenitril (NSN), sind wichtige Intermediate unter anderem zur Herstellung der sogenannten Nikotinate, wie Nikotinsäureamid (NSA) und Nikotinsäure (NS). NSA und NS werden beispielsweise Nahrungsmitteln als Vitamine zugesetzt oder als Bausteine für die Herstellung pharmazeutisch wirksamer Verbindungen verwendet.

Es ist bekannt, dass Verbindungen der Formeln I bis III schrittweise über die Säureamide der Formeln 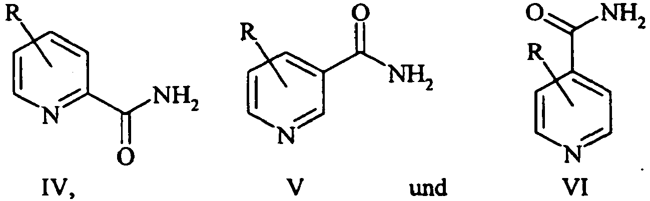 worin R wie oben definiert ist, zu den Säuren der Formel 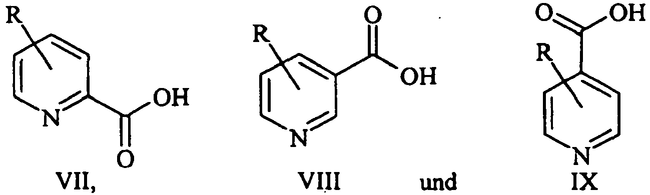 worin R wie oben definiert ist, hydrolysiert werden können.

In den bekannten Verfahren zur Herstellung von NSA und NS aus NSN entstehen jedoch hohe Salzfrachten im Produktionsablauf und stossen daher zunehmend auf Akzeptanzprobleme. Seit einiger Zeit wachsen die Anforderungen an die Reduzierung der Salzfracht in Produktionsabläufen, da sich die Beseitigung solcher Abwässer, beispielsweise durch Verbrennung oder Verklappung zunehmend verteuert. Weiterhin lassen sich die Salze nur aufwendig aus den teilweise ebenfalls in Salzform vorliegenden Produkten entfernen. Diese Salzfracht ist insbesondere bei NSA und NS problematisch wenn diese als Ernährungs- oder Futtermittelzusätze verwendet werden. Bei höheren Temperaturen steigt die Hydrolysegeschwindigkeit stark an, jedoch zersetzten sich NSA und NS bei höheren Temperaturen und bilden die entsprechenden Pyridine, die durch eine negative Geruchsnote die Verwendung der entstehenden Säuren und Amide und in der Futtermittelindustrie einschränken.

Gemäss US 5756750 worin Ammoniak als bevorzugte Base genannt wird, kann die Hydrolyse von NSN bei niedriger Natronlaugekonzentration (14 Teile Base zu 100 Teilen NSN) zu NSA und bei äquimolarer Natronlaugekonzentration zu NS ablaufen. Nach Neutralisation mit HCl fäfllt etwa ein Teil NaCl pro 2 Teile NS als Abfallprodukt an. Eine weitere Möglichkeit zur Darstellung von NS aus NSN ist die enzymatische Hydrolyse. Die dabei eingesetzten Nitrilasen benötigen meist komplexe Medienzusammensetzungen. Weiterhin liefern diese Enzyme geringe Raum-Zeit-Ausbeuten und erfordern ein Aussalzen der NS mit daraus resultierenden Salzanfall (Process Biochemistry 2006, 41(9), 2078-2081 und JP 2005176639). Im enzymatischen Verfahren ist weiterhin oftmals ein Aktivitätsverlust der Enzyme festzustellen (Journal of Molecular Catalysis B: Enzymatic 2006, 39(1-4), 55-58).

Der Artikel "Hydrolysis of nitriles. IV. Continuous hydrolysis of 3-cyanopyridine to nicotinamide and nicotinic acid", STN CAPLUS, 1973, XP002127619 beschreibt ein Verfahren zur kontinuierlichen Hydrolyse von 3-cyanopyridin in Gegenwart von Ammoniak. Die Reaktion wird bei 200-250°C, einer Verweilzeit von 90-100 min sowie einem Druck von 4.6 MPa durchgeführt.

Aufgabe der vorliegenden Erfindung war daher ein Verfahren zu entwickeln, das sich durch eine deutliche Reduzierung der Salzfrachten bei hohen Raum-Zeit-Ausbeuten auszeichnet. Weiterhin sollte da Produkt weitgehend frei von Pyridinderivaten sein.

Diese Aufgabe wurde entsprechend Anspruch 1 gelöst.

Beansprucht wird ein Verfahren zur Hydrolyse von Verbindungen der Formeln 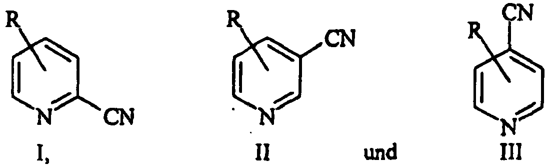 worin R Wasserstoff oder C₁₀₋₂₀-Alkyl ist,
zu den entsprechenden Amiden und/oder Säuren in wässrigen Fluiden, bei einer Temperatur von mindestens 100 °C, vorzugsweise mindestens 150 °C, besonders bevorzugt von mindestens 200 °C, und einem Druck von mindestens 10 MPa, vorzugsweise von mindestens 15 MPa, besonders bevorzugt von mindestens 20 MPa, in Gegenwart von Ammoniak in einer konzentration von mindestens 0,3 mol/l.

Je nach Reaktionsführung werden Gemische unterschiedlicher Zusammensetzung erhalten.

Die erste Stufe, die Hydrolyse der Nitrile, erfolgt unter den erfindungsgemässen Bedingungen schneller als die zweite Stufe, die Hydrolyse der Amide. Bei geeigneter Reaktionskontrolle kann die Hydrolyse überwiegend auf der Stufe der Amide der Formeln IV bis VI angehalten oder bis zur nächsten Stufe der entsprechenden Säuren der Formeln VII bis IX weitergeführt werden. In den unten angerührten Beispielen wird gezeigt, dass die Produktzusammensetzung durch Wahl geeigneter Parameter (Temperatur, Druck, Katalysatorart und -menge) über einen grossen Bereich gesteuert werden kann. Dies ist insbesondere von Vorteil für die Verbindungen NSA und NS, welche im Emährungs- und Futtermittelbereich auch als Gemische verwendet werden können, und bei denen das Produkt - nach Abtrennung der gegebenenfalls verwendeten Lösungsmittel und Katalysatoren- direkt weiterverwendet werden kann.

Zweckmässig wird im erfindungsgemässen Verfahren für die bevorzugte Herstellung von Verbindungen der Formeln IV bis IX eine Temperatur von höchstens 280 °C vorzugsweise von höchstens 270 °C, besonders bevorzugt von höchstens 250 °C nicht überschritten. Oberhalb 400 °C findet fast quantitativ die Decarboxylierung der Säuren der Formeln VII bis IX zu den entsprechenden Pyridinderivaten statt. In einer bevorzugten Verfahrensvariante wird die Reaktion kontinuierlich in einem Strömungsreaktor mit mindestens einer Reaktionszone durchgeführt.

Geeignete Reaktoren sind beispielsweise Strömungsreaktoren mit oder ohne statische Mischer oder druckfeste Mikroreaktoren mit oder ohne separate Mischzonen. Bei der Zuführung von homogenen Gemischen kann auf besondere Mischeinrichtungen verzichtet werden. Bei getrennter Zuführung von Edukten und wässriger Phase ist eine Mischeinrichtung vorteilhaft. Unter den Mikroreaktoren sind insbesondere solche auf Metallbasis geeignet, die sich schnell und genau temperieren lassen. Gegebenenfalls enthalten geeignete Strömungsreaktoren zusätzliche Zuführungen für die Einspeisung von Katalysatoren entlang der Reaktionszone. Diese zusätzliche Einspeisung ist insbesondere für die Einleitung von gasförmigem Ammoniak bei basischer Reaktionsführung vorteilhaft.

Geeignete Strömungsreaktoren weisen gegebenenfalls eine oder mehrere Nachreaktionszonen auf.

In einem statischen Mischer beträgt die Verweildauer in der Reaktionszone vorzugsweise zwischen 0,1 und 7000 Sekunden, besonders bevorzugt zwischen 30 und 6000 Sekunden.

Unter wässrigen Fluiden werden im vorliegenden Verfahren Wasser enthaltende Lösungsmittel, insbesondere wässrige Flüssigkeiten, verstanden, in welchen die übrigen Lösungsmittelbestandteile inert gegen über den Ausgangsverbindungen, den Produkten sind und sich unter den Reaktionsbedingungen nicht zersetzen. Als Lösungsmittelbestandteile kommen neben Wasser auch organische Verbindungen wie C₁₋₁₀-Alkohole, C₁₋₁₀-Carbonsäuren, Ester der vorgenannten Alkohole und Carbonsäuren, Methyl-*tert*-butylketon und Paraffine in Betracht. Die genannten Carbonsäuren können hierbei, insbesondere bei den hohen Reaktionstemperaturen, gleichzeitig als Lösungsmittel und als Katalysator wirken. Vorzugsweise besteht das Fluid überwiegend aus Wasser.

In einer bevorzugten Verfahrensvariante enthält das wässrige Fluid zu Beginn der Reaktion ein Nitril der Formel I, II oder III in einer Menge von 0,05 bis 30 Gew.-%, besonders bevorzugt von 10 bis 20 Gew.-% In einer weiteren bevorzugten Variante wird das Verfahren bei einem Druck im Bereich von mindestens 10 MPa, vorzugsweise von 20 bis 60 MPa, besonders bevorzugt von 20 bis 35 MPa durchgeführt.

Generell überwiegt mit steigender Temperatur bzw. mit steigender Verweilzeit im Reaktor die Bildung der Säuren der Formeln VII bis IX gegenüber der Bildung der Amide der Formeln IV bis VI. Wird das Verfahren in einem wässrigen Fluid bei nicht vorgegebenem pH durchgeführt - also ohne Zusatz von Säuren oder Basen -, lässt sich das Produktverhältnis der Säuren zu den Amiden durch Temperatur und Druck in einem weiten Bereich steuern. Die Reaktionszeiten unter diesen Bedingungen sind jedoch relativ lange und begünstigen die Decarboxylierung der gebildeten Nikotinsäure zu den entsprechenden Pyridinderivaten.

Zur Erhöhung der Reaktionsgeschwindigkeit und zur weiteren Steuerung der Selektivität wird im erfindungsgemässen Verfahren Ammoniak zugesetzt. Der Katalysator kann auch die Bildung von Nebenprodukten reduzieren.

Bei Verwendung von Ammoniak wird neben einer allgemeinen Steigerung der Reaktionsgeschwindigkeit die Selektivität hinsichtlich der Bildung der Säuren der Formeln VII bis IX verbessert. Bei vergleichbaren Temperaturen und Reaktionszeiten hat die Verwendung von Ammoniak gegenüber dem Verfahren unter Verwendung saurer Katalysatoren oder in Abwesenheit von Katalysatoren den weiteren Vorteil einer reduzierten Decarboxylierung.

Der Katalysator wird in einer Menge von mindestens 0,3 mol/L verwendet.

Insbesondere bei Konzentrationen unterhalb 0,3 mol/L steigt die Tendenz zur Decarboxylierung der NS zu Pyridinderivaten.

Da die Reaktion unter Druck stattfindet, besteht die Möglichkeit Ammoniak unter Druck zuzusetzen. Besonders bevorzugt wird Ammoniak als gasförmige Base verwendet. In einer ganz besonders bevorzugten Ausführungsform wird in Gegenwart eines basischen Katalysators zusätzlich gasförmiges Ammoniak unter Druck zugegeben. Ammoniak als basischer Katalysator ist besonders vorteilhaft, da Ammoniak beispielsweise im Vakuum bequem aus dem Produkt entfernt werden kann und somit keine Neutralisation erfordert.

Bei der zweistufigen Hydrolyse von Nitrilen über die entsprechenden Säureamide zu den Säuren ist der zweite Schritt geschwindigkeitsbestimmend. Wie die Beispiele 1 bis 4 zeigen, nimmt die Nitrilkonzentration, insbesondere in Anwesenheit eines Katalysators, deutlich schneller ab als die Säurekonzentration ansteigt. Durch den Unterschied der Hydrolysegeschwindigkeiten kann abhängig von den Reaktionsparametern das Verhältnis der Amide zu den Säuren in einem weiten Bereich gesteuert werden. Die Hydrolyse von der Nitrilen zu den Amiden verläuft dabei vollständig und unter den Reaktionsbedingungen irreversibel, während die Hydrolyse der Amide zu den entsprechenden Säuren, insbesondere bei Verwendung von Ammoniak als basischem Katalysator, einem Reaktionsgleichgewicht unterliegt. Weiterhin kann die Hydrolyse der Amide unter den erfindungsgemässen Bedingungen auch durchgeführt werden, wenn die Amide auf anderem Wege synthetisiert oder die Nitrile auf anderem Wege separat hydrolysiert wurden. Die Amide lassen sich beispielsweise chemisch auch bei Normaldruck und Normaltemperatur aus den Nitrilen durch Hydrolyse erhalten. Die Amide lassen sich aber auch biotechnologisch durch enzymatische Hydrolyse der Nitrile erhalten, wie beispielsweise offenbart in WO-A 99/05306.

Der erste Schritt im erfindungsgemässen Verfahren, also die Hydrolyse der Nitrile zu den Amiden kann auch drucklos beziehungsweise ohne zusätzliche Druckbeaufschlagung erfolgen.

Ein weiterer Aspekt der vorliegenden Erfindung ist daher die Hydrolyse von Amiden der Formeln 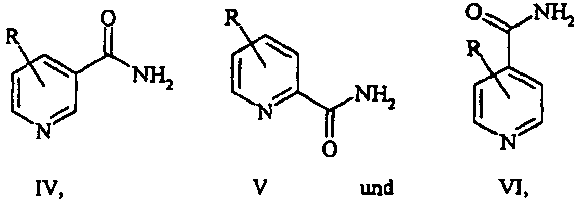 worin R Wasserstoff oder C₁₋₂₀-Alkyl ist zu den entsprechenden Säuren, bei einer Temperatur von mindestens 100 °C, vorzugsweise mindestens 150 °C, besonders bevorzugt von mindestens 200 °C, und einem Druck von mindestens 10 MPa, vorzugsweise von mindestens 15 MPa, besonders bevorzugt von mindestens 20 MPa, in genwart von Ammoniak in einer Konzentration von mindestens 0,3 mol/L. Zweckmässig wird für die Hydrolyse der Amide eine Temperatur von 280 °C, vorzugsweise von 270 °C, besonders bevorzugt von 250 °C nicht überschritten.

In einer bevorzugten Verfahrensvariante wird die Reaktion kontinuierlich in einem Strömungsreaktor durchgeführt. Geeignete Reaktoren sind beispielsweise Strömungsreaktoren mit oder ohne statische Mischer oder druckfeste Mikroreaktoren mit oder separate Mischzonen. Bei der Zuführung von homogenen Gemischen kann auf besondere Mischeinrichtungen verzichtet werden. Bei getrennter Zuführung von Edukten und wässriger Phase ist eine Mischeinrichtung vorteilhaft. Unter den Mikroreaktoren sind insbesondere solche auf Metallbasis geeignet, die sich schnell und genau temperieren lassen. Gegebenenfalls enthalten geeignete Strömungsreaktoren zusätzliche Zuführungen für die Einspeisung von Katalysatoren entlang der Reaktionszone. Diese zusätzliche Einspeisung ist insbesondere für die Einleitung von gasförmigem Ammoniak bei basischer Reaktionsführung vorteilhaft.

Geeignete Strömungsreaktoren weisen mindestens eine Reaktionszone und gegebenenfalls eine oder mehrere Nachreaktionszonen auf.

In einem statischen Mischer beträgt die Verweildauer in der Reaktionszone vorzugsweise zwischen 10 und 7000 Sekunden, besonders bevorzugt zwischen 30 und 6000 Sekunden.

Unter wässrigen Fluiden werden im vorliegenden Verfahren Wasser enthaltende Lösungsmittel, insbesondere wässrige Flüssigkeiten, verstanden, in welchen die übrigen Lösungsmittelbestandteile inert gegen über den Ausgangsverbindungen, den Produkten sind und sich unter den Reaktionsbedingungen nicht zersetzen. Als Lösungsmittelbestandteile kommen neben Wasser auch organische Verbindungen wie C₁₋₁₀-Alkohole, C₁₋₁₀-Carbonsäuren, Ester der vorgenannten Alkoholen und Carbonsäuren, Methyl-*tert-*butylketon und Paraffine in Betracht. Die genannten Carbonsäuren können hierbei, insbesondere bei den hohen Reaktionstemperaturen, gleichzeitig als Lösungsmittel und als Katalysator wirken. Vorzugsweise besteht das Fluid überwiegend aus Wasser.

In einer bevorzugten Verfahrensvariante enthält das wässrige Fluid zu Beginn der Reaktion ein Amid der Formel IV, V oder VI in einer Menge von 0,05 bis 30 Gew.-%, besonders bevorzugt von 10 bis 20 Gew.-%

In einer weiteren bevorzugten Variante wird das Verfahren bei einem Druck im Bereich von mindestens 10 bis höchstens 60 MPa, vorzugsweise von 20 bis 60 MPa, besonders bevorzugt von 20 bis 35 MPa durchgeführt.

Zur Erhöhung der Reaktionsgeschwindigkeit und zur Steuerung der Selektivität wird im erfindungsgemässen Verfahren Ammoniak zugesetzt Der Katalysator kann auch die Bildung von Nebenprodukten reduzieren.

Die Verwendung von Ammoniak bedingt eine allgemeine Steigerung der Reaktionsgeschwindigkeit. Bei vergleichbaren Temperaturen und Reaktionszeiten hat die Verwendung von Ammoniak gegenüber dem Verfahren unter Verwendung saurer Katalysatoren oder in Abwesenheit von Katalysatoren den weiteren Vorteil einer reduzierten Decarboxylierung.

Der Katatysator wird in einer Menge von mindestens 0,3 mol/L verwendet.

Da die Reaktion unter Druck stattfindet, besteht die Möglichkeit Ammoniak unter Druck zuzusetzen. Besonders bevorzugt wird Ammoniak als gasförmige Base verwendet. In einer ganz besonders bevorzugten Ausführungsform wird in Gegenwart eines basischen Katalysators zusätzlich gasförmiges Ammoniak unter Druck zugegeben. Ammoniak als basischer Katalysator ist besonders vorteilhaft, da Ammoniak beispielsweise im Vakuum bequem aus dem Produkt entfernt werden kann und somit keine Neutralisation erfordert.

### Beispiele:

Die folgenden Beispiele verdeutlichen die Ausführung der Erfindung, ohne dass darin eine Einschränkung zu sehen ist.

In den Ergebnistabellen der Beispiele 1-4 werden in den einzelnen Spalten die Werte für die Reaktionsdauer τ in [s], für die Konzentration des Nitrils zu verschiedenen Reaktionszeiten c_{CN} in [mM], für die Konzentration der gebildeten Säure zu verschiedenen Reaktionszeiten c_{COOH} in [mM], für die Konzentration des gebildeten Amids zu verschiedenen Reaktionszeiten c_{CONH2} in [mM], für den Umsatz des Nitrils U_{CN} in [%], Für den Flächenanteil der gebildeten Säure A_{COOH} in [%], für den Flächenanteil des gebildeten Amids A_{CONH2} in %, für die Selektivität der gebildeten Säure S_{COOH} in [%] sowie für die Selektivität des gebildeten Amids A_{CONH2} in [%] angegeben. In den Beispielen 5 bis 8 und den Vergleichsbeispielen 1 bis 3 sind abweichend davon die Konzentration des eingesetzten Amids c_{CONH2} in [M], die Konzentration der erhaltenen Säure c_{COOH} in [M] sowie für die Selektivität von Pyridin
S_{Py} in [%] angegeben.
n.b. = nicht bestimmt

### Beispiel 1 nicht erfindungsgemäß: (Tabellen 1 und 2) Hydrolyse von 0,5 Gew.-% Nikotinsäurenitril in reinem Wasser

### Beispiel 2 nicht erfindungsgemäß: (Tabellen 3 und 4) Hydrolyse von 0,5 Gew.-% Nikotinsäurenitril in 10 mmol Schwefelsäure

### Beispiel 3: (Tabellen 5 bis 10) Hydrolyse von 0,5 Gew.-% Nikotinsäurenitril in verschieden konzentrierten Ammoniaklösungen

### Beispiel 4: (Tabellen 11 bis 14) Hydrolyse von 5, 10 und 15 Gew.-% Nikotinsäurenitril in konz. Ammoniak (14,7 M, 25%)

### Beispiel 1: Hydrolyse von 0,5 % Nikoktinonitril in reinem Wasser

**Tabelle 1: Ergebnisse bei 200 °C und 25 MPa in Wasser**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 96,9 | 0 | 0 | 0 | 0 | 0 | | |
| 50 | 94,6 | 0 | 0,85 | 2,3 | 0 | 0,9 | 0 | 38 |
| 72 | 93,6 | 0,06 | 1,33 | 3,4 | 0,1 | 1,4 | 1,8 | 40,5 |
| 101 | 92,3 | 0,13 | 1,82 | 4,7 | 0,1 | 1,9 | 2,9 | 40 |
| 206 | 87,3 | 0,14 | 3,59 | 9,9 | 0,1 | 3,7 | 1,5 | 38 |
| 269 | 84,2 | 0,15 | 4,74 | 13,0 | 0,2 | 4,9 | 1,2 | 37,5 |

**Tabelle 2: Ergebnisse bei 250 °C und 25 MPa in Wasser**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 83,9 | 0,12 | 0 | 0 | 0 | 0 | | |
| 59 | 76,9 | 0,32 | 5,57 | 8,3 | 0,2 | 6,6 | 2,88 | 80,4 |
| 74 | 75,3 | 0,35 | 7,30 | 10,2 | 0,3 | 8,7 | 2,73 | 85,6 |
| 101 | 73,6 | 0,41 | 8,80 | 12,2 | 0,3 | 10,5 | 2,81 | 85,9 |
| 195 | 68,3 | 0,65 | 12,68 | 18,6 | 0,6 | 15,1 | 3,37 | 81,3 |
| 325 | 63,6 | 0,84 | 15,59 | 24,2 | 0,9 | 18,6 | 3,54 | 76,8 |
| 390 | 61,2 | 0,97 | 16,76 | 27,0 | 1,0 | 20,0 | 3,75 | 74,1 |

### Beispiel 2: Hydrolyse von und 0,5 Gew.-% Nikotinsäurenitril mit Schwefelsäure

**Tabelle 3: Ergebnisse bei 250 °C und 25 MPa in 10 mM Schwefelsäure**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 50,5 | 0 | 0 | 0 | 0 | 0 | | |
| 45 | 48,7 | 0,27 | 0,87 | 3,4 | 0,5 | 1,7 | 15,50 | 50,0 |
| 72 | 47,7 | 0,87 | 1,37 | 5,5 | 1,7 | 2,7 | 31,66 | 49,5 |
| 98 | 46,9 | 1,34 | 1,73 | 7,0 | 2,7 | 3,4 | 37,89 | 48,9 |
| 180 | 44,2 | 2,76 | 2,93 | 12,5 | 5,5 | 5,8 | 43,69 | 46,5 |
| 255 | 41,8 | 3,90 | 3,93 | 17,2 | 7,7 | 7,8 | 44,95 | 45,4 |
| 379 | 39,3 | 4,71 | 4,87 | 22,2 | 9,3 | 9,6 | 42,02 | 43,5 |

**Tabelle 4: Ergebnisse bei 250 °C und 30 MPa in 10 mM Schwefelsäure**

| T | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 41,1 | 0 | 0 | 0 | 0 | 0 | | |
| 47 | 39,6 | 0,3 | 0,9 | 3,7 | 0,7 | 2,1 | 17,8 | 58,3 |
| 75 | 38,5 | 0,8 | 1,4 | 6,2 | 2,0 | 3,5 | 32,0 | 55,9 |
| 100 | 37,3 | 1,4 | 2,0 | 9,1 | 3,5 | 4,9 | 37,9 | 54,1 |
| 182 | 34,6 | 3,2 | 3,2 | 15,9 | 7,7 | 7,9 | 48,4 | 49,8 |
| 278 | 32,3 | 4,6 | 4,1 | 21,4 | 11,3 | 10,0 | 52,6 | 46,8 |
| 385 | 30,1 | 5,9 | 5,0 | 26,6 | 14,4 | 12,2 | 53,9 | 45,7 |
| 883 | 22,0 | 10,4 | 8,1 | 46,5 | 25,3 | 19,7 | 54,4 | 42,4 |

### Beispiel 3: Hydrolyse von und 0,5 Gew.-% Nikotinsäurenitril in verschieden konzentrierten Ammoniaklösungen

**Tabelle 5: Ergebnisse bei 250 °C und 30 MPa in 5 mM Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 43,93 | 0 | 0,00 | 0 | 0 | 0 | | |
| 56,2 | 22,04 | 1,08 | 19,92 | 49,8 | 2,5 | 45,3 | 4,94 | 91,0 |
| 97,6 | 18,24 | 2,03 | 22,93 | 58,5 | 4,6 | 52,2 | 7,89 | 89,3 |
| 182,6 | 12,29 | 3,92 | 26,96 | 72,0 | 8,9 | 61,4 | 12,39 | 85,2 |
| 286,3 | 8,01 | 6,08 | 29,10 | 81,8 | 13,8 | 66,2 | 16,94 | 81,0 |
| 421,3 | 4,76 | 9,13 | 29,04 | 89,2 | 20,8 | 66,1 | 23,30 | 74,1 |
| 549,5 | 2,85 | 11,69 | 28,03 | 93,5 | 26,6 | 63,8 | 28,47 | 68,2 |
| 712,1 | 1,35 | 15,61 | 26,15 | 96,9 | 35,5 | 59,5 | 36,67 | 61,4 |

**Tabelle 6: Ergebnisse bei 250 °C und 30 MPa in 50 mM Ammoniak**

| T | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 39,33 | 0 | 0 | 0 | 0 | 0 | | |
| 49 | 8,01 | 1,55 | 28,16 | 79,6 | 4,0 | 71,6 | 4,96 | 89,9 |
| 74 | 6,42 | 2,37 | 28,84 | 83,7 | 6,0 | 73,3 | 7,19 | 87,6 |
| 102,3 | 5,15 | 3,38 | 29,17 | 86,9 | 8,6 | 74,2 | 9,89 | 85,4 |
| 190,6 | 2,70 | 6,56 | 29,00 | 93,1 | 16,7 | 73,7 | 17,90 | 79,2 |
| 296,1 | 1,43 | 9,67 | 27,56 | 96,4 | 24,6 | 70,1 | 25,50 | 72,7 |
| 379,5 | 0,87 | 12,17 | 26,29 | 97,8 | 30,9 | 66,8 | 31,64 | 68,4 |
| 539,3 | 0,16 | 16,09 | 22,93 | 99,6 | 40,9 | 58,3 | 41,07 | 58,5 |

**Tabelle 7: Ergebnisse bei 250 °C und 30 MPa in 1,17 M Ammoniak**

| T | c_{CN} | c_{COOH} | c_{CONH2} | c_{CN} | A_{COOH} | A_{CONH2} | S_{COOH} | S_{CONH2} |
|---|---|---|---|---|---|---|---|---|
| 0 | 35,30 | 0 | 0,57 | 0 | 0 | 0 | | |
| 45,9 | 0,25 | 4,73 | 30,25 | 99,3 | 13,4 | 84,1 | 13,51 | 84,7 |
| 73,4 | 0 | 7,40 | 27,21 | 100,0 | 21,0 | 75,5 | 20,95 | 75,5 |
| 99,8 | 0 | 9,77 | 25,08 | 100,0 | 27,7 | 69,4 | 27,67 | 69,4 |
| 171,9 | 0 | 14,71 | 20,53 | 100,0 | 41,7 | 56,6 | 41,68 | 56,6 |
| 292,0 | 0 | 18,46 | 16,58 | 100,0 | 52,3 | 45,4 | 52,29 | 45,4 |
| 446,9 | 0 | 21,34 | 13,28 | 100,0 | 60,5 | 36,0 | 60,46 | 36,0 |

**Tabelle 8: Ergebnisse bei 250 °C und 30 MPa in 5,88 M Ammoniak**

| τ | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 38,30 | 0 | 0,23 | 0 | 0 | 0 | | |
| 44,8 | 0 | 10,40 | 25,22 | 100,0 | 27,2 | 65,2 | 27,15 | 65,2 |
| 74,7 | 0 | 17,17 | 21,08 | 100,0 | 44,8 | 54,4 | 44,81 | 54,4 |
| 104,4 | 0 | 21,11 | 17,31 | 100,0 | 55,1 | 44,6 | 55,12 | 44,6 |
| 188,4 | 0 | 26,89 | 11,54 | 100,0 | 70,2 | 29,5 | 70,21 | 29,5 |
| 291,3 | 0 | 30,59 | 7,48 | 100,0 | 79,9 | 18,9 | 79,87 | 18,9 |
| 464,7 | 0 | 32,67 | 4,05 | 100,0 | 85,3 | 10,0 | 85,28 | 10,0 |
| 679,5 | 0 | 33,76 | 1,74 | 100,0 | 88,1 | 3,9 | 88,13 | 3,9 |

**Tabelle 9: Messdaten und Ergebnisse bei 250 °C und 30 MPa in 14,7 M Ammoniak**

| T | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 37,03 | 0 | 3,15 | 0 | 0 | 0 | | |
| 43,9 | 0 | 8,86 | 30,18 | 100 | 22,0 | 73, | 23,91 | 73 |
| 118,7 | 0 | 21,36 | 18,31 | 100 | 53,2 | 40,9 | 57,68 | 40,9 |
| 225,5 | 0 | 29,54 | 9,59 | 100 | 73,5 | 17,4 | 79,77 | 17,4 |
| 343,3 | 0 | 33,6 | 5,50 | 100 | 83,6 | 6,3 | 90,72 | 6,3 |
| 561,8 | 0 | 36,1 | 3,15 | 100 | 89,8 | 0 | 97,48 | 0 |

**Tabelle 10: Messdaten und Ergebnisse bei 280 °C und 30 MPa in 5,88 M Ammoniak**

| T | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 38,3 | 0 | 0 | 0 | 0 | 0 | | |
| 53,5 | 0 | 14,9 | 23,1 | 100 | 39 | 60 | 39 | 60 |
| 77,0 | 0 | 19,4 | 18,4 | 100 | 51 | 48 | 51 | 48 |
| 103,3 | 0 | 22 | 15,4 | 100 | 57 | 40 | 57 | 40 |
| 190,0 | 0 | 26,9 | 9,6 | 100 | 70 | 25 | 70 | 25 |
| 303,3 | 0 | 30,4 | 5,1 | 100 | 79 | 13 | 79 | 13 |
| 545,6 | 0 | 33,2 | 1,7 | 100 | 87 | 4 | 87 | 4 |

| | | | | | | | | |
|---|---|---|---|---|---|---|---|---|
| Pyridinmenge nach 545,6 s kleiner 0,1%. | | | | | | | | |

### Beispiel 4: Hydrolyse von 5, 10 und 15 Gew.-% Nikotinsäurenitril in konz. Ammoniak (14,7 M, 25%)

**Tabelle 11: Ergebnisse bei 250 °C und 30 MPa in 14,7 M Ammoniak und 5 Gew.-% Nikotinsäurenitril**

| T | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 366 | 0 | 28 | 0 | 0 | 0 | | |
| 117 | 26 | 112 | 257 | 93 | 31 | 62 | 33 | 67 |
| 238 | 0 | 200 | 193 | 100 | 55 | 45 | 55 | 45 |
| 590 | 0 | 294 | 85 | 100 | 80 | 15 | 80 | 15 |
| 801 | 0 | 314 | 61 | 100 | 86 | 9 | 86 | 9 |

**Tabelle 12: Ergebnisse bei 250 °C und 30 MPa in 14,7 M Ammoniak und 10 Gew.-% Nikotinsäurenitril**

| T | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 791 | 0 | 57 | 0 | 0 | 0 | | |
| 47 | 211 | 57 | 576 | 73 | 7 | 66 | 10 | 89 |
| 117 | 72 | 177 | 596 | 91 | 22 | 68 | 25 | 75 |
| 223 | 20 | 294 | 513 | 97 | 37 | 58 | 38 | 59 |
| 576 | 0 | 526 | 311 | 100 | 67 | 32 | 67 | 32 |
| 817 | 0 | 586 | 225 | 100 | 74 | 21 | 74 | 21 |

**Tabelle 13: Ergebnisse bei 250 °C und 30 MPa in 14,7 M Ammoniak und 15 Gew.-% Nikotinsäurenitril**

| T | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 1109 | 0 | 75 | 0 | 0 | 0 | | |
| 123 | 199 | 97 | 887 | 82 | 9 | 73 | 11 | 89 |
| 219 | 76 | 240 | 867 | 93 | 22 | 71 | 23 | 77 |
| 574 | 0 | 610 | 571 | 100 | 55 | 45 | 55 | 45 |
| 852 | 0 | 731 | 451 | 100 | 66 | 34 | 66 | 34 |

**Tabelle 14: Ergebnisse bei 280 °C und 30 MPa in 14,7 M Ammoniak und 10 Gew.-% Nikotinsäurenitril**

| T | c_{CN} | c_{COOH} | c_{CONH2} | U_{CN} | A_{COOH} | A_{CONH2} | S_{COOH} | S_{CONH2} |
|---|---|---|---|---|---|---|---|---|
| 0 | 1038 | 0 | 81 | 0 | 0 | 0 | | |
| 51 | 232 | 63 | 824 | 78 | 6 | 72 | 8 | 92 |
| 106 | 119 | 158 | 842 | 89 | 15 | 73 | 17 | 83 |
| 312 | 0 | 386 | 659 | 100 | 37 | 56 | 37 | 56 |
| 505 | 0 | 542 | 554 | 100 | 52 | 46 | 52 | 46 |
| 699 | 0 | 599 | 493 | 100 | 58 | 40 | 58 | 40 |

| | | | | | | | | |
|---|---|---|---|---|---|---|---|---|
| Pyridinmenge nach 699 s kleiner 0,1%. | | | | | | | | |

Bei allen Umsetzungen der Beispiele 1 bis 4 unter Druck und bei Temperaturen unter 280 °C, sowie der folgenden Beispiele 5 bis 8 konnte bei Reaktionsende kein Pyridin im Produkt festgestellt werden.

Die Beispiele 5 bis 8 und Vergleichsbeispiele wurden in einem Rohreaktor mit Nikotinamid als Ausgangsverbindung durchgeführt.

### Beispiel 5: Ergebnisse bei 150 °C und 30 MPa in 9,28 M Ammoniak.

| T | c_{CONH2} | c_{COOH} | U_{CONH2} |
|---|---|---|---|
| 0 | 1,056 | 0,053 | 0,0 |
| 284 | 0,993 | 0,116 | 6,0 |
| 533 | 0,948 | 0,161 | 10,3 |
| 801 | 0,926 | 0,183 | 12,3 |
| 893 | 0,894 | 0,214 | 15,3 |
| 1102 | 0,868 | 0,241 | 17,8 |
| 1385 | 0,836 | 0,272 | 20,8 |
| 2699 | 0,727 | 0,382 | 31,2 |

### Beispiel 6: Ergebnisse bei 250 °C und 30 MPa in 1,21 M Ammoniak.

| T | c_{CONH2} | c_{COOH} | U_{CONH2} |
|---|---|---|---|
| 0 | 1,147 | 0,042 | 0,0 |
| 247 | 0,651 | 0,538 | 43,2 |
| 520 | 0,432 | 0,757 | 62,3 |
| 699 | 0,336 | 0,854 | 70,7 |
| 840 | 0,277 | 0,912 | 75,8 |
| 1021 | 0,211 | 0,978 | 81,6 |
| 1203 | 0,202 | 0,987 | 82,4 |
| 1694 | 0,133 | 1,056 | 88,4 |
| 2365 | 0,121 | 1,068 | 89,4 |

### Beispiel 7: Ergebnisse bei 200 °C und 30 MPa in 1,21 M Ammoniak.

| τ | c_{CONH2} | c_{COOH} | U_{CONH2} |
|---|---|---|---|
| 0 | 1,148 | 0,041 | 0,0 |
| 264 | 0,968 | 0,221 | 15,7 |
| 544 | 0,843 | 0,346 | 26,5 |
| 791 | 0,759 | 0,430 | 33,9 |
| 916 | 0,715 | 0,475 | 37,7 |
| 1095 | 0,649 | 0,541 | 43,5 |
| 1330 | 0,569 | 0,621 | 50,4 |
| 1841 | 0,369 | 0,820 | 67,8 |
| 2636 | 0,341 | 0,849 | 70,3 |

### Beispiel 8: Ergebnisse bei 200 °C und 10 MPa in 0,3 M Ammoniak.

| τ | c_{CONH2} | c_{COOH} | U_{CONH2} |
|---|---|---|---|
| 0 | 1,168 | 0,026 | 0,0 |
| 1263 | 0,568 | 0,626 | 51,3 |
| 2192 | 0,332 | 0,862 | 71,6 |
| 5017 | 0,117 | 1,078 | 90,0 |

In den Vergleichsbeispielen wurde Nikotinsäureamid zur Untersuchung der Kinetik des zweiten Reaktionsschrittes umgesetzt. Angegeben ist die Selektivität gebildeten Pyridins in Mol% gegenüber dem ausgangsmaterial und der gebildeten Säure.

### Vergleichsbeispiel 1: Ergebnisse bei 250 °C und 5 MPa in 0,3 M Ammoniak.

| T | c_{CONH2} | c_{COOH} | S_{Py} | U_{CONH2} | S_{CONH2} | S_{COOH} |
|---|---|---|---|---|---|---|
| 0 | 1,173 | 0,017 | 0,0 | 0,0 | 100,0 | 0,0 |
| 1234 | 0,555 | 0,635 | 0,0 | 52,6 | 47,4 | 52,6 |
| 2206 | 0,353 | 0,836 | 0,0 | 69,9 | 30,1 | 69,9 |
| 4960 | 0,131 | 1,046 | 1,2 | 88,9 | 11,1 | 87,7 |

### Vergleichsbeispiel 2: Ergebnisse bei 250 °C und 6 MPa in 0,3 M Ammoniak.

| T | c_{CONH2} | c_{COOH} | S_{Py} | U_{CONH2} | S_{CONH2} | S_{COOH} |
|---|---|---|---|---|---|---|
| 0 | 1,169 | 0,021 | 0,0 | 0,0 | 100,0 | 0,0 |
| 1246 | 0,569 | 0,621 | 0,1 | 51,3 | 48,7 | 51,3 |
| 2324 | 0,310 | 0,876 | 0,3 | 73,5 | 26,5 | 73,2 |
| 4999 | 0,119 | 1,057 | 1,2 | 89,8 | 10,2 | 88,6 |

### Vergleichsbeispiel 3: Ergebnisse bei 250 °C und 30 MPa in 0,1 M Ammoniak.

| τ | c_{CONH2} | c_{COOH} | S_{Py} | U_{CONH2} | S_{CONH2} | S_{COOH} |
|---|---|---|---|---|---|---|
| 0 | 1,151 | 0,041 | 0 | 0 | 100,0 | 0 |
| 1691 | 0,515 | 0,677 | 0,0 | 55,3 | 44,7 | 55,3 |
| 2298 | 0,392 | 0,800 | 0,0 | 65,9 | 34,1 | 65,9 |
| 5236 | 0,102 | 1,055 | 3,1 | 91,2 | 8,8 | 88,1 |

## Patentansprüche

1. Ein Verfahren zur Hydrolyse von Verbindungen der Formeln 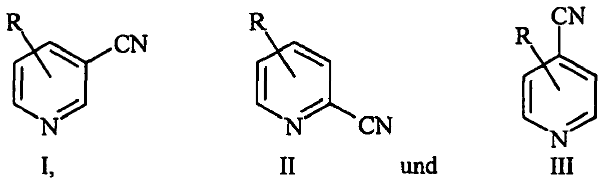 worin R Wasserstoff oder C₁₋₂₀-Alkyl ist,
zu den entsprechenden Amiden und/oder Säuren in wässrigen Fluiden, **dadurch gekennzeichnet, dass** die Reaktion bei einer Temperatur von mindestens 100 °C und einem Druck von mindestens 10 MPa, vorzugsweise von mindestens 15 MPa, besonders bevorzugt von mindestens 20 MPa, in Gegenwart von Ammoniak, durchgeführt wird, wobei das Ammoniak in einer Konzentration von mindestens 0,3 mol/L verwendet wird.

2. Verfahren nach Anspruch 1, **dadurch gekennzeichnet, dass** die Reaktion kontinuierlich in einem Strömungsreaktor mit mindestens einer Reaktionszone durchgeführt wird.

3. Verfahren nach Anspruch 1 oder 2, **dadurch gekennzeichnet, dass** das wässrige Fluid zu Beginn der Reaktion ein Nitril der Formel I, II oder III in einer Konzentration von 0,05 bis 30 Gew.-% enthält.

4. Ein Verfahren zur Hydrolyse von Verbindungen der Formeln 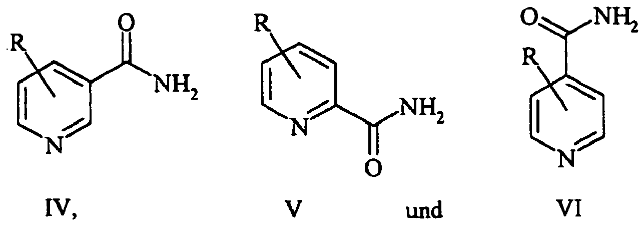 worin R Wasserstoff oder C₁₋₂₀-Alkyl ist, zu den entsprechenden Säuren in wässrigen Fluiden, **dadurch gekennzeichnet, dass** die Reaktion bei einer Temperatur von mindestens 100 °C und einem Druck von mindestens 10 MPa, vorzugsweise von mindestens 15 MPa, besonders bevorzugt von mindestens 20 MPa, in Gegenwart von Ammoniak, durchgeführt wird, wobei das Ammoniak in einer Konzentration von mindestens 0,3 mol/L vorliegt.

5. Verfahren nach Anspruch 4, **dadurch gekennzeichnet, dass** die Reaktion kontinuierlich in einem Strömungsreaktor mit mindestens einer Reaktionszone durchgeführt wird.

6. Verfahren nach Anspruch 4 oder 5, **dadurch gekennzeichnet, dass** das wässrige Fluid zu Beginn der Reaktion ein Amid der Formel IV, V oder VI in einer Konzentration von 0,05 bis 30 Gew.-% enthält.

## Claims

1. Process for the hydrolysis of compounds of the formulae 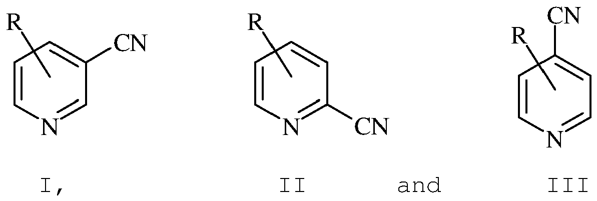 in which R is hydrogen or C₁₋₂₀-alkyl,
to give the corresponding amides and/or acids in aqueous fluids, **characterized in that** the reaction is carried out at a temperature of at least 100°C and a pressure of at least 10 MPa, preferably of at least 15 MPa, particularly preferably of at least 20 MPa, in the presence of the ammonia being used in a concentration of at least 0.3 mol/l.

2. Process according to Claim 1, **characterized in that** the reaction is carried out continuously in a flow reactor with at least one reaction region.

3. Process according to Claim 1 or 2, **characterized in that** the aqueous fluid at the beginning of the reaction comprises a nitrile of the formula I, II or III in a concentration of 0.05 to 30% by weight.

4. Process for the hydrolysis of compounds of the formulae 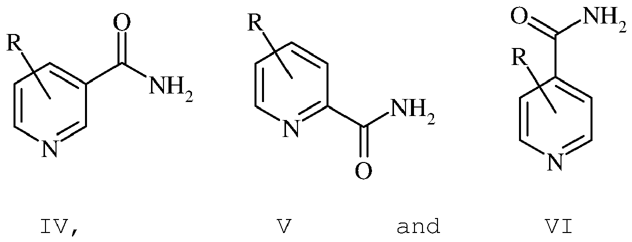 in which R is hydrogen or C₁₋₂₀-alkyl,
to give the corresponding acids in aqueous fluids, **characterized in that** the reaction is carried out at a temperature of at least 100°C and a pressure of at least 10 MPa, preferably of at least 15 MPa, particularly preferably of at least 20 MPa, in the presence of the ammonia being present in a concentration of at least 0.3 mol/l.

5. Process according to Claim 4, **characterized in that** the reaction is carried out continuously in a flow reactor with at least one reaction region.

6. Process according to Claim 4 or 5, **characterized in that** the aqueous fluid at the beginning of the reaction comprises an amide of the formula IV, V or VI in a concentration of 0.05 to 30% by weight.

## Revendications

1. Procédé d'hydrolyse de composés des formules 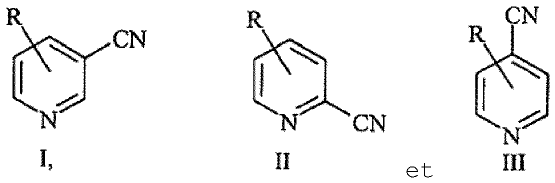 dans lesquelles R est l'hydrogène ou un alkyle en C₁₋₂₀, pour former les amides et/ou acides correspondants dans des fluides aqueux, **caractérisé en ce que** la réaction est réalisée à une température d'au moins 100 °C et à une pression d'au moins 10 MPa, de préférence d'au moins 15 MPa, de manière particulièrement préférée d'au moins 20 MPa, en présence d'ammoniac, l'ammoniac étant utilisé en une concentration d'au moins 0,3 mol/L.

2. Procédé selon la revendication 1, **caractérisé en ce que** la réaction est réalisée en continu dans un réacteur en écoulement comprenant au moins une zone de réaction.

3. Procédé selon la revendication 1 ou 2, **caractérisé en ce que** le fluide aqueux contient au début de la réaction un nitrile de formule I, II ou III en une concentration de 0,05 à 30 % en poids.

4. Procédé d'hydrolyse de composés des formules 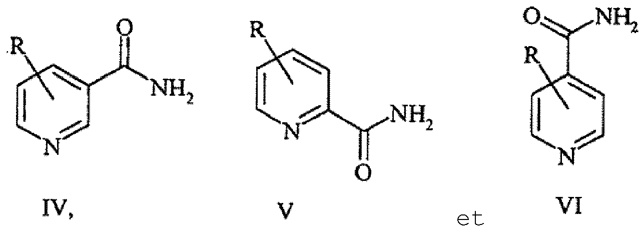 dans lesquelles R est l'hydrogène ou un alkyle en C₁₋₂₀, pour former les acides correspondants dans des fluides aqueux, **caractérisé en ce que** la réaction est réalisée à une température d'au moins 100 °C et à une pression d'au moins 10 MPa, de préférence d'au moins 15 MPa, de manière particulièrement préférée d'au moins 20 MPa, en présence d'ammoniac, l'ammoniac étant présent en une concentration d'au moins 0,3 mol/L.

5. Procédé selon la revendication 4, **caractérisé en ce que** la réaction est réalisée en continu dans un réacteur en écoulement comprenant au moins une zone de réaction.

6. Procédé selon la revendication 4 ou 5, **caractérisé en ce que** le fluide aqueux contient au début de la réaction un amide de formule IV, V ou VI en une concentration de 0,05 à 30 % en poids.
